Europäisches Patentamt

European Patent Office

Office européen des brevets

(19)

(11) Veröffentlichungsnummer: **0 381 042**

**A1**

(12) **EUROPÄISCHE PATENTANMELDUNG**

(21) Anmeldenummer: **90101452.2**

(22) Anmeldetag: **25.01.90**

(51) Int. Cl.⁵: **A61M 25/00, A61M 25/10**

(30) Priorität: **02.02.89 DE 3903119**

(43) Veröffentlichungstag der Anmeldung:
**08.08.90 Patentblatt 90/32**

(84) Benannte Vertragsstaaten:
**AT DE ES FR GB IT**

(71) Anmelder: **WILLY RÜSCH AG**
**Willy-Rüsch-Strasse 4-10**
**D-7053 Kernen/Rommelshausen(DE)**

(72) Erfinder: **Korth, Knut, Dr.**
**c/o Lorettokrankenhaus, Mercystrasse 6-14**
**D-7800 Freiburg(DE)**

(74) Vertreter: **Patentanwälte Kohler - Schwindling**
**- Späth**
**Hohentwielstrasse 41**
**D-7000 Stuttgart 1(DE)**

(54) **Katheter.**

(57) Die Erfindung betrifft einen Katheter mit mehreren Lumina (1,2,5) und zum Drainagelumen führenden Öffnungen (3,7,9) an der Katheterspitze. Sie besteht darin, daß der Querschnitt jeder Abflußöffnung (9) kleiner als der Querschnitt des Lumens (1) ist, zu dem die Öffnung (9) führt und das der Drainage dient. Dadurch kann dann, wenn im Urin feste Gewebeteile enthalten sind, dieser zuverlässig abgeführt werden.

EP 0 381 042 A1

# Katheter

Die Erfindung bezieht sich auf einen Katheter mit mindestens einem Lumen (Kanal) und zu diesen führenden Öffnungen (Augen) an der Katheterspitze.

Bei bekannten Kathetern sind diese Augen meist relativ groß, ihr Durchmesser entspricht häufig dem Durchmesser des Lumens, zu dem diese Augen führen. Der Außendurchmesser eines Katheters ist in der Regel durch den Anwendungszweck des Katheters bestimmt, beispielsweise bei einem transurethralen Katheter durch die lichte Weite der Harnröhre, bei postoperativen Kathetern beispielsweise durch die Gegebenheiten der Blasendrainage und dergleichen. Der Außendurchmesser eines Katheters ist eine von vorneherein feststehende Größe. Von dieser Größe hängt wiederum der Querschnitt des Lumens ab, bei einem einläufigen Katheter entspricht der Durchmesser des Lumens dem Außendurchmesser minus der Wandstärke, beispielsweise kann bei einem einläufigen Katheter aus Gummi oder Kunststoff, der einen Außendurchmesser von ca. 8 mm aufweist, der Durchmesser des Lumens etwa 5 mm betragen. Ballonkatheter weisen mindestens 2 Lumina auf, bei denen ein zweites Lumen zum Aufblasen des in der Nahe der Katheterspitze befindlichen Ballons dient, mit dem der Katheter im Körper festgehalten wird oder aber auf die Operationswunde eine Kompression ausgeübt wird. Postoperative Ballonkatheter sind im allgemeinen dreiläufig, ein Lumen dient zur Ableitung des Urins und der durch die Operation bedingten Sekrete und Blutkoagel, ein Lumen führt zu dem Ballon und ein drittes Lumen dient zum Einleiten einer Spülflüssigkeit in den Drainagebereich. Da, wie oben erwähnt, der Außendurchmesser eines Katheters eine vom Anwendung szweck her bestimmte Größe hat, ist der Maximaldurchmesser der einzelnen Lumen davon abhängig, wieviele Lumina in dem Katheter Platz finden müssen. Die Große der Augen wurde bei der Herstellung von bekannten Kathetern nicht auf den Lumendurchmesser abgestimmt, sondern möglichst groß gewählt.

Postoperative Katheter, insbesondere transurethrale Harnblasenkatheter, weisen im allgemeinen an ihrer Spitze zwischen ein und vier Katheteraugen (Drainagelöcher) auf, deren Querschnitt annähernd dem des Katheterlumens entspricht. Die bekannten postoperativen Katheter, die die Wundsekrete und den Urin abführen sollen, neigen jedoch zu Verstopfungen, wenn feste Blutkoagel oder aber sogar Resektionsschnipsel beigemischt sind. Diese festen Fremdkörper werden durch die großen Katheteraugen in das relativ kleine Katheterlumen hineingesogen und verschließen die Augen und erst recht das Lumen. Insbesondere treten derartige Verstopfungen bei Kathetern auf, deren Augen sich gegenüber liegen. Die Gewebeteilchen fädeln sich in beide einander gegenüberliegende Augen zugleich ein. Infolge der gleichmäßigen Dikke bekannter Verweilkatheter drainiert ohnehin nur das am weitesten augen liegende Katheterauge die Wunde, eine Verstopfung dieses Auges mit einem in das Lumen hängenden Gewebeteilchen reicht daher aus, um die Drainage fast vollständig zu unterbinden. Diese Schwierigkeiten treten insbesondere nach transurethralen Operationen in der Blase oder an der Prostata auf.

Der Erfindung liegt die Aufgabe zugrunde, einen Katheter zu entwickeln, der auch dann, wenn im Urin feste Gewebeteile enthalten sind, diesen zuverlässig abzuführen.

Diese Aufgabe wird gemäß der Erfindung dadurch gelöst, daß der Durchmesser jeder Öffnung kleiner als der Durchmesser des Lumen ist, zu dem die Öffnung führt. Dadurch werden Fremdkörper, die die Öffnung oder das Lumen verstopfen konnten, bereits vor deren Eintritt abgewiesen. Jedes Teilchen aber, das die Katheteröffnung passiert hat, wird unbehindert auch durch das Katheterlumen abgeführt, weil dieses ja einen größeren Querschnitt als das Katheterauge aufweist.

Kleine Öffnungen sind zwar durch das Patent 1 196 327 bekannt, diese führen jedoch nicht zu einem Lumen und dienen daher nicht zur Drainage.

Bei einer Ausführungsform der Erfindung verteilen sich mehrere Öffnungen über einen Abschnitt von 3 bis 5 cm an der Katheter spitze. Auch hier erfolgt die hauptsächliche Drainage durch die am weitesten außen liegenden Öffnungen. Die vielen zur Katheterspitze hin zusätzlich vorgesehenen Öffnungen bieten jedoch eine Sicherheit dafür, daß wenn der Eingang zu den Öffnungen durch größere Blutgerinnsel oder Gewebeteilchen zugesetzt ist, weiter innen liegende Öffnungen die Drainage übernehmen.

Bei Ausführungsformen der Erfindung können über einen 3 bis 5 cm langen Abschnitt an der Katheterspitze 30 bis 40 zu dem drainierenden Lumen führende Öffnungen verteilt sein.

Bei einer Ausführungsform des erfindungsgemäßen Katheters aus Gummi oder einem elastischen Kunststoff beträgt der Durchmesser der Öffnungen ca. 1,5 mm bei einer lichten Weite des Lumens von ca. 5 mm im Durchmesser.

Die Erfindung kann bei allen verschiedenen bekannten Spitzenformen verwirklicht sein (z.B. Nelaton, Tiemann, Mercier).

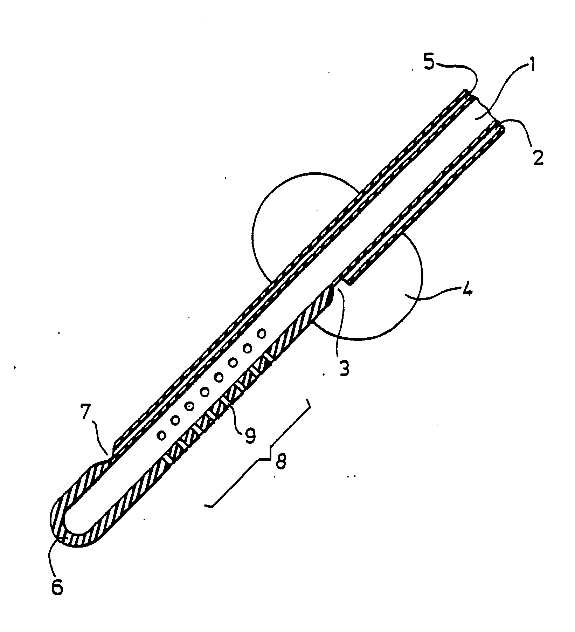

Weitere Merkmale der Erfindung ergeben sich aus den Ansprüchen in Verbindung mit der folgenden Beschreibung und der Zeichnung. Die einzelnen Merkmale können je einzeln für sich oder zu mehreren bei Ausführungsformen der Erfindung verwirklicht sein.

In der Zeichnung ist eine Ausführungsform der Spitze eines erfindungsgemäßen Katheters im Schnitt dargestellt.

Bei der in der Zeichnung dargestellten Ausführungsform ist ein postoperativer Verweilkatheter dreiläufig. Er weist ein Lumen 1 mit einem verhältnismäßig großen Durchmesser auf, ein zweites Lumen 2 mit kleinem Durchmesser, das in eine Öffnung 3 innerhalb eines Ballons 4 mündet und zum Füllen des Ballons dient. Ein drittes Lumen 5 weist ebenfalls einen verhältnismäßig kleinen Durchmesser auf, es mündet unmittelbar an der Spitze 6 in eine seitliche Öffnung 7 und dient zur Zuführung einer Spülflüssigkeit. Auf einem ca. 3 bis 5 cm langen Abschnitt 8 sind in das Lumen 1 führende Abfließöffnungen 9 angeordnet, die über die erwähnte Länge und über den Umfang dieses Katheterabschnittes 8 verteilt sind mit Ausnahme des Bereiches, in dem das Lumen 5 zur Spülöffnung 7 führt. In dem Abschnitt 8 sind ca. 30 bis 40 Öffnungen 9 vorgesehen. Die Öffnungen weisen einen Durchmesser von ca. 1,5 mm auf, der Durchmesser des Lumens 1 beträgt im dargestellten Beispiel ca. 6 mm.

Die Spülöffnung 7 des zur Spülung dienenden Lumens 5 läßt sich in verschiedenen Höhen der Katheterspitze anbringen. Der Katheter kann beispielsweise zwischen 14 und 26 Charr. dick sein.

Bei der transurethralen Anwendung des Katheters kann der Ballon 4 so angeordnet und geformt sein, daß er nach einer Prostataoperation die Operationsloge komprimiert und sie von der Blase her abschließt. Oder aber der Ballon 4 kann relativ klein ausgebildet sein und lediglich zum Festhalten des Katheters in der Blase benutzt werden.

Der erfindungsgemäße Katheter kann aus einem weicheren Werkstoff hergestellt sein als die bekannten Spülkatheter, z.B. aus einem weichen Gummi mit einem silikonhaltigen Überzug, weil eine manuelle Aspiration mit Hilfe einer Spritze, die zur Beseitigung von Koageln dient, nicht erforderlich ist. Im übrigen kann die Katheterspitze mit Ausnahme einer großkalibrigen Öffnung alle bekannten Formen aufweisen und die Ballongrößen können dem jeweiligen Verwendungszweck optimal angepaßt sein.

## Ansprüche

1. Katheter mit mehreren Lumina (1, 2, 5) und zu diesen fuhrenden Öffnungen (3, 7, 9) an der Katheterspitze, dadurch gekennzeichnet, daß der Querschnitt jeder Abflußöffnung (9) kleiner als der Querschnitt des Lumens (1) ist, zu dem die Öffnung (9) führt und die der Drainage dient.

2. Katheter nach Anspruch 1, dadurch gekennzeichnet, daß die Drainageöffnungen (9) über einen an die Katheterspitze (6) sich anschließenden, 3 bis 5 cm langen Katheterabschnitt (8) verteilt angeordnet sind.

3. Katheter nach Anspruch 2, dadurch gekennzeichnet, daß in dem Abschnitt (8) des Katheters zwanzig bis fünfzig Drainageöffnungen (9) vorgesehen sind.

4. Katheter nach einem der vorhergehenden Ansprüche, dadurch gekennzeichnet, daß bei einer lichten Weite des der Drainage dienenden Lumens (1) zwischen 2 und 7 mm der Durchmesser der Öffnungen (9) ca. 1-2 mm (vorzugsweise 1,5 mm) beträgt.

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl⁵) |
|---|---|---|---|
| X | FR - E - 87 564 (R.FLEURY) * Gesamt; insbesondere Fig. 4,5 * | 1 | A 61 M 25/00 A 61 M 25/10 |
| A | -- | 2-4 | |
| X | US - A - 4 752 286 (Y.OKADA) * Fig. 1-3; Spalte 2, Zeilen 37-55 * -- | 1 | |
| A | -- | 2,4 | |
| X | GB - A - 1 513 918 (INT.PAPER COMP.) * Fig. 2; Seite 5, Zeilen 5-13; Patentanspruch 1 * | 1 | |
| A | -- | 4 | |
| A | DE - A1 - 2 435 627 (DOW CORNING) * Fig. 1-3; Seite 4, letzter Absatz * -- | 1 | |
| A | US - A - 4 642 092 (G.MOSS) * Fig. 1,3; Spalte 3, Zeile 49 - Spalte 4, Zeile 64 * ---- | 1 | |

**RECHERCHIERTE SACHGEBIETE (Int Cl⁵)**

A 61 M 1/00
A 61 M 25/00
A 61 M 27/00

Der vorliegende Recherchenbericht wurde für alle Patentansprüche erstellt.

| Recherchenort WIEN | Abschlußdatum der Recherche 28-05-1990 | Prüfer LUDWIG |
|---|---|---|